# EUROPEAN PATENT APPLICATION

(11) **EP 3 662 822 A1**
(43) Date of publication of application: **10.06.2020**
(21) Application number: 18000964.9
(22) Date of filing: 12.12.2018
(51) Int. Cl.: A61B 5/024, A61B 5/053, A61B 5/16, G07C 13/00

(54) **DEVICE TO PERFORM PSYCHOLOGICAL TESTS**

(30) Priority: 03.12.2018 PL 42802018
(71) Applicant: Politechnika Slaska, 44-100 Gliwice (PL)
(72) Inventor: Dobrowolska, Malgorzata, 40-540 Katowice (PL); Gzik, Marek, 44-100 Gliwice (PL); Wodarski, Piotr, 43-190 Mikolow (PL); Jedrasiak, Karol, 44-190 Mikolow (PL); Nawrat, Aleksander, 41-807 Zabrze (PL); Szabo, Stanislav, 04441Sady Nad TOrysou (SK); Vajdova, Iveta, 04013 Kosice (SK)

(57) **Abstract**

A device for psychological tests and quantitative assessment comprises a base with conductance sensors mounted thereon, pulse and a token presence optical sensor, characterized in that on a base (1) is located a box (2) for automatic response counting, and the base comprises a plurality of points (4) where measuring rings (5) are attached thereto and a measuring ring (6), whereas the measuring rings (5 and 6) each comprises at least one sensor (7) and/or (8) for measuring skin conductance in a wired or wireless way, further the device comprises a wireless pulse sensor (9) located between the measuring ring (5) and the measuring ring (6), whereas the pulse sensor (9) is located on a straight line linking the measuring rings attachment points (4), and on continuation of the straight line linking the measuring rings attachment points (4) in the box (2) is located an inlet (10) automatically sucking in RFID tokens with responses, additionally the inlet (10) is provided with the scales that weighs the token and checks on the accuracy thereof.

## Description

The subject of the invention is a device to perform psychological tests and quantitative assessment, used in psychological practices.

In the psychological practices there are no devices that would improve calculation of responses obtained from respondents whilst analysing emotional state and stress level of the respondent. So far, so called psychological quantitative assessment commonly performed by psychologists in diagnosis using questionnaires, psychological tests, scales and paper-and-pencil checklists have been administered by means of computer software, printed films to calculate scores or manually. Emotional state and stress level, however, have been assessed by observation, an interview with a respondent or using additional quantitative methods.

Similar solutions have been applied, among others, in the devices "Multifunctional wrist strap for physical examination" known, for example, from the invention description no. CN206587057, "Intellectualized medical care data control system" CN201019738, as well as in the device "Traditional Chinese medicine pulse condition monitoring wrist-strap type device with function of RFID" CN203169156. The overview of prior art shows that those present in the market serve mainly medical purposes and do not comprise a combination of sensors used in the present solution to perform psychological tests and quantitative assessment. The prior art solutions comprise mainly the sensors in a hand strap wore in a wrist area and sensors located in separate places on a device. Such solutions are not used to perform psychological tests and quantitative assessment, they do not allow to measure the respondent's emotional state at the same time. Thus, those solutions do not allow to optimize time and costs of examinations.

There are no devices to calculate scores and observation at the same time. The present solution allows to reduce time of assessment with regard to prior art examination methods. Modularity of the device allows to administer both individual and group examinations, which optimizes time and costs of assessments. Additional technical advantage of the device construction is fraud prevention by no possibility to insert more than one token at a time and by verification of coherence between the response and emotional state of the respondent.

The object of the invention is a device for psychological tests and quantitative assessment comprising a base with conductance, pulse sensors located thereon and with an optical sensor of token presence. On the base is located a box for automatic response counting, and the base comprises a plurality of attachment points where measuring rings are attached thereto, whereas the measuring rings comprise at least one sensor for skin conductance measure in a wired or wireless way, further the device comprises a wireless pulse sensor. On continuation of the straight line linking the measuring rings attachment points in the box is located an inlet automatically sucking in the RFID tokens with the responses, additionally the inlet is provided with the scales that weighs the token and checks on the accuracy thereof.

In the present solution, a response to a questionnaire is given by inserting a relevant token into the device, read in the device in a wireless way.

The device for psychological tests and quantitative assessment comprises a base with sensors located thereon and a box for automatic response counting. The device comprises measuring rings with a sensor to measure skin conductance in a wired or wireless way The device is also provided with a wireless pulse sensor. The present device allows to measure emotional state and stress level at the same time (by measuring the skin conductance and heart rhythm) and counting tokens for each test item of the diagnostic tool in order to obtain immediate score interpretation for the three aforementioned aspects: emotional state, stress level and substantive diagnostic tool.

The present device is of a universal character, serves for assessment of so called normal people as well as with psychological disorders, evaluated on the basis of the emotional state diagnosis and its co-relation with responses given. Additionally, it can be used for all existing, validated questionnaire diagnostic tools (checklists, tests, scales, etc.), according to the assessment methodology on the market of psychological services. An advantage of the device, apart from optimization of the very process of psychological assessment, is additionally that the assessment process is interesting, which has an impact on decreasing the level of fatigue at the assessment with assessment tools currently present on the market. An additional objective of the present solution is facilitation of response interpretation by attractive for respondents way of administration, where instead of uniform text with questions numbered in order and written on a piece of paper with possible responses: yes - don't know - no a respondent has a set of single questions in a form of tokens provided with transmitting-receiving aerials in RFID (Radio-Frequency IDentification) technology.

Each token can take different shapes depending on a group being assessed. For children, those may be natural symbols, or strips for adolescents and adults that are inserted one by one to the box. Additionally, the device automatically counts responses on a basis of RFID wireless token detection (Radio-frequency identification) located in the device during assessment and allows to verify coherence of the responses with the pulse values measured during the assessment and the skin conductivity measured during the assessment.

The subject of the invention is shown in the preferred embodiment in the drawing where Fig. 1 shows a front view of the device, Fig. 2 side view, Fig. 3 top view, and Fig. 4 axonometric view.

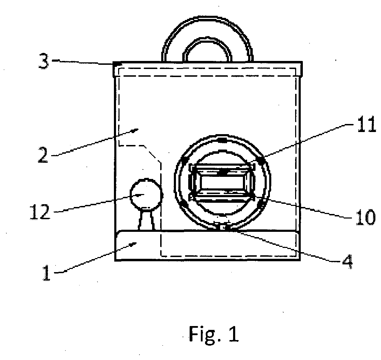

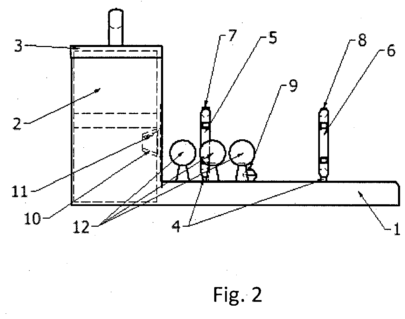

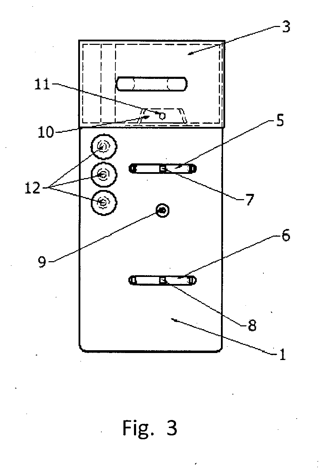

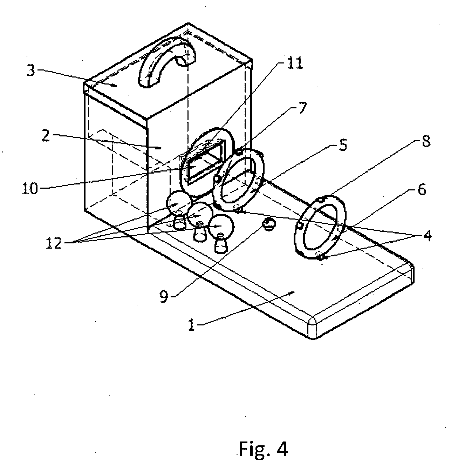

The present solution according to Figs. 1, 2, 3 and 4 comprises a base 1 with a box 2 located thereon for questionnaire response automatic counting, and the box comprises a cover 3 to remove RFID tokens with responses. Measuring rings 5 and 6 each comprises at least one sensor 7 and/or 8 for measuring skin conductance (measured in units: micro simens µS) in a wired or wireless way. The sensors 7 and 8 are located on the rings 5 and 6 within a constant distance from adjacent sensors. The present device comprises also a wireless pulse sensor 9 located between the nearer measuring ring 5 and the further measuring ring 6. The pulse sensor 9 is located on a straight line linking the measuring rings attachment points 4. Pulse measuring is conducted in a wireless way by the optoelectronic method, with the optoelectronic transducers the illuminance of a beam of infrared light reflected from the limb is measured. The pulse measurement is within the range from 30 to 400 beats per minute. On continuation of the straight line linking the measuring rings attachment points 4 in the box 2 is located an inlet 10 automatically sucking in the RFID tokens with the responses. The inlet 10 is provided with the scales that weighs the token and checks on the accuracy thereof. The RFID token presence near the inlet 10 is detected by an optic sensor 11 located at the upper part of the inlet 10. Feedback and indication of response coherence are provided by diodes 12 located on the base 1.

The construction of the device enables conversion of information coming from the RFID tokens being inserted and information from the measuring sensors 7, 8 and 9. Giving the response to a questionnaire question correctly consists in picking the relevant RFID token with the response and placing it in the box 2. The placing is performed by putting the limb with the token through the measuring rings 5 and 6 over the pulse wireless measuring sensor 9. At the moment when the token is nearing the inlet 10 opening it is detected by the optic sensor 11, which activates the mechanism of automatic token suction into the box 2. At the moment when the RFID token has been located in the box 2 the value representing the response to the question is read wirelessly. At the moment when the limb of the respondent is in the measuring nearer ring 5 or further ring 6 the measuring of the skin resistance is performed within each of the rings. When the limb is over the pulse measuring sensor 9 the pulse is measured. The information from the sensors 7, 8 and 9 and the responses to the questions read from the RFID tokens are transmitted to the counting module of the device. The termination of the calculation and measuring is presented using the interface comprising at least three diodes 12 lighting in different colours.

## Claims

1. A device for psychological tests and quantitative assessment comprises a base with conductance sensors mounted thereon, pulse and a token presence optical sensor, **characterized in that** on a base (1) is located a box (2) for automatic response counting, and the base comprises a plurality of points (4) where measuring rings (5) are attached thereto and a measuring ring (6), whereas the measuring rings (5 and 6) each comprises at least one sensor (7) and/or (8) for measuring skin conductance in a wired or wireless way, further the device comprises a wireless pulse sensor (9) located between the measuring ring (5) and the measuring ring (6), whereas the pulse sensor (9) is located on a straight line linking the measuring rings attachment points (4), and on continuation of the straight line linking the measuring rings attachment points (4) in the box (2) is located an inlet (10) automatically sucking in RFID tokens with responses, additionally the inlet (10) is provided with the scales that weighs the token and checks on the accuracy thereof.

2. The device according to claim 1, **characterized in that** the box comprises a cover (3).

3. The device according to claim 1, **characterized in that** the pulse sensor is an optoelectronic transducer, whereas pulse measuring is conducted in a wireless way.

4. The device according to claim 1, **characterized in that** it comprises a plurality of measuring rings (5 and 6) that perform skin conductance measuring for a limb placed inside the ring.

5. The device according to claim 1, **characterized in that** the measuring rings attachment points (4) and the localization of the pulse measuring sensor (9) are situated in one straight line.

6. The device according to claim 1, **characterized in that** the skin conductance measuring is performed with the wired or wireless skin conductance sensor (7 and 8).

7. The device according to claim 1, **characterized in that** the sensors (7 and 8) are located on the rings (5 and 6), in a circle within a constant distance from each other.

8. The device according to claim 1, **characterized in that** at the inlet (10) to the box (2) is located the RFID token presence check optical sensor (11).

9. The device according to claim 1, **characterized in that** the base (1) comprises mounted diodes (12) signalling coherence between the response and emotional state of the respondent.

10. The device according to claim 1, **characterized in that** it provides wireless reading of the responses to questionnaire questions for the RFID tokens placed in the part of the box (2).
